# EUROPEAN PATENT APPLICATION

(11) **EP 4 241 766 A1**
(43) Date of publication of application: **13.09.2023**
(21) Application number: 22160583.5
(22) Date of filing: 07.03.2022
(51) Int. Cl.: A61K 31/00, A61K 31/122, A61P 35/00, G01N 33/50

(54) **USE OF OLFACTORY RECEPTOR OR13A1 LIGANDS IN THE TREATMENT OF LYMPHOMAS**

(71) Applicant: Fondazione Per L'Istituto Oncologico Di Ricerca (IOR), 6500 Bellinzona (CH); Fondazione per l'Istituto di Ricerca in Biomedicina (IRB), 6500 Bellinzona (CH)
(72) Inventor: SARTORI, Giulio, 6500 Bellinzona (CH); CAVALLI, Andrea, 8047 Zürich (CH); CASCIONE, Luciano, 21016 Luino (IT); MATKOVIC, Milos, 6500 Bellinzona (CH); BERTONI, Francesco, 6500 Bellinzona (CH)
(74) Representative: M. Zardi & Co S.A.

(57) **Abstract**

The invention is directed to the use of ligands of the olfactory receptor OR13A1 for the treatment or prevention of lymphomas. The invention is based on the finding that the olfactory receptor OR13A1 is strongly expressed in lymphoma forms such as diffuse large B cells lymphoma (DLBCL) and mantle cell lymphoma (MCL): this receptor was found not merely characteristic but markedly pro-oncogenic and responsive to stimulation by agonist and antagonist ligands. This allows for the first time to establish a clear rationale for targeting this receptor in the treatment of lymphomas such as DLBCL and MCL. The invention also relates to methods to identify candidate patients for an improved treatment of lymphomas based on their level of cell expression of the OR13A1 receptor. Further included is a method to select a compound useful for the treatment or prevention of lymphomas including the step of assaying its ligand activity of the olfactory receptor OR13A1.

## Description

### STATE OF THE ART

Lymphomas are among the most common cancers in adults and especially in adolescents and young adults [1, 2]. Despite the huge improvements achieved in the last decades, still too many patients succumb due to their disease [3-5] and strong efforts are therefore still required to develop efficient novel therapeutic targets. Examples are diffuse large B cell lymphoma (DLBCL) and mantle cell lymphoma (MCL). DLBCL is the most common type in in adults and young adults, accounting for over 30% of the cases [2, 6]. The cornerstone of first line therapies for DLBCL patients is still represented by chemotherapy. In adults the standard treatment is represented by the R-CHOP regimen (rituximab, cyclophosphamide, doxorubicin, vincristine and prednisone), but 40% or more of patients are not cured [6]. MCL accounts for 5-10% of all lymphomas in adults [7]. The introduction of high dose cytarabine-containing chemoimmunotherapeutic regimens and autologous transplantation has considerably improved the outcome of young/fit MCL patients, nonetheless, approximately 20 to 25% of MCL patients demonstrate inadequate efficacy of intensified chemoimmunotherapy as they are either primary refractory or relapse within two years from autologous stem cell transplantation [7].

G protein-coupled receptors (GPCRs) are the largest family of integral membrane proteins in the human genome and mediate important cellular responses to endogenous (amines, cations, lipids, peptides and glycoproteins) and exogenous (light, tastants and odorants) ligands and stimuli. All GPCRs contain an extracellular N-terminus and a cytosolic C-terminus separated by seven transmembrane α-helices connected by three intracellular and three extracellular peptide loops. Activation of GPCRs by ligands induces conformational changes of the receptor, promoting the coupling with G proteins, which modulate the downstream signaling cascades. Altered signaling pathways associated with GPCRs are implicated in the pathophysiology of various diseases, including cancer, infections and metabolic, immunological or neurodegenerative disorders [8]. Expression and functional alterations of chemokine receptors (CXCR3, CXCR4 and CXCR5), sphingosine-1-phospate receptors (S1PR1, S1PR2 and S1PR3), or purinergic receptor GPR34 have been reported in different lymphoma types [9, 10].

The olfactory receptors family comprises over 700 GPCRs, which, albeit initially identified and characterized for their expression in olfactory cells, can be expressed in different tissues [11], including cancers [12-25]. Examples are OR51E1, also known as Prostate-Specific G Protein-Coupled Receptor 2 (PSGR2) due to its expression in prostate cancer cells [16-18, 25], OR2B6, OR2T6 and OR2W3 in breast cancer [19-21], and OR51E2 in melanoma cells [22, 23].Olfactory receptor OR13A1 (also identified as murine orthologue Olfr211) is a protein that in humans is encoded by the *OR13A1* gene (sequence description: Gene ID: 79290, cf. https://www.ncbi.nlm.nih.gov/gene?Db=gene&Cmd=DetailsSearch&Term=7929 0#gene-expression

A large group of at least thirty potential genetic markers has been used in the double-hit expression signature in diffuse large B cell lymphoma (DLBCL) (Ennishi et al. J.Clin. Oncol., 190 Volume 37, Issue 3 pp. 190-201 2019, also published with the patent application WO2020079591A1): the group included, among others, the olfactory receptor OR13A1, but no focus or suggestion was present in the publication as to the possible pro-oncogenic role and/or therapeutic relevance of this particular receptor alone, i.e. isolated from the remaining markers; in fact, this double-hit expression signature requires the use of multiple gene markers in combination, to highlight a possibly pathologic condition.

The complex landscape of olfactory receptors, which vary in types and levels of expression among different tumours, also with different quali/quantitative response to their stimulation, makes it difficult to conceive strategies of tumour treatments based thereon. Although some improvements have been made in the treatment of lymphoma, still too many patients die for this disease and novel targets and treatments are needed. In particular, since several forms of this disease exist with different symptoms, course and outcome, there is still a highly unmet need in this area for strongly effective, patient-tailored medicaments.

### SUMMARY OF THE INVENTION

It has been found that the olfactory receptor OR13A1 is strongly expressed in lymphomas: this receptor was found not merely characteristic of these cells but having a marked pro-oncogenic role; this allows for the first time to establish a clear rationale for targeting this receptor in the treatment of lymphomas. The invention is accordingly directed to the use of ligands of the olfactory receptor OR13A1 for the treatment or prevention of lymphomas, in particular DLBCL or MCL. The invention also relates to methods to identify candidate patients for an improved treatment of lymphomas based on their level of cell expression of the OR13A1 receptor. Further part of the invention is a method to select a compound useful for the treatment or prevention of lymphomas including the step of assaying its ligand activity of the olfactory receptor OR13A1. The experiments in the description show the successful reduction of lymphoma cell viability in presence of the test ligand of OR13A1 cyclohexanone. Experimental silencing of the receptor abolished this effect, confirming its OR13A1-specificity.

### DESCRIPTION OF THE FIGURES

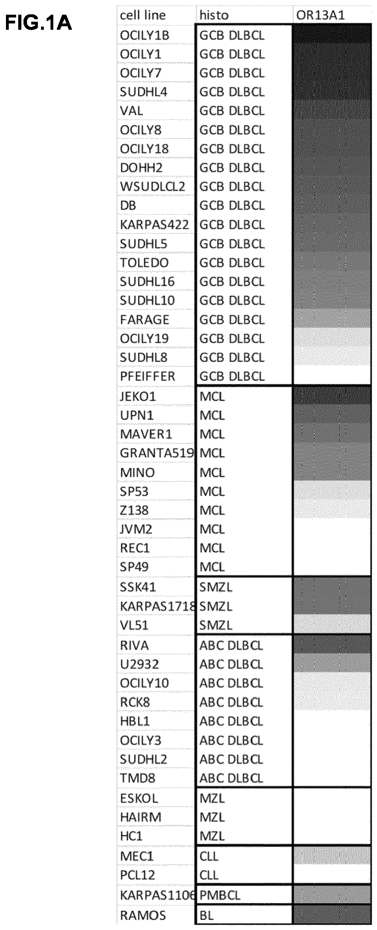

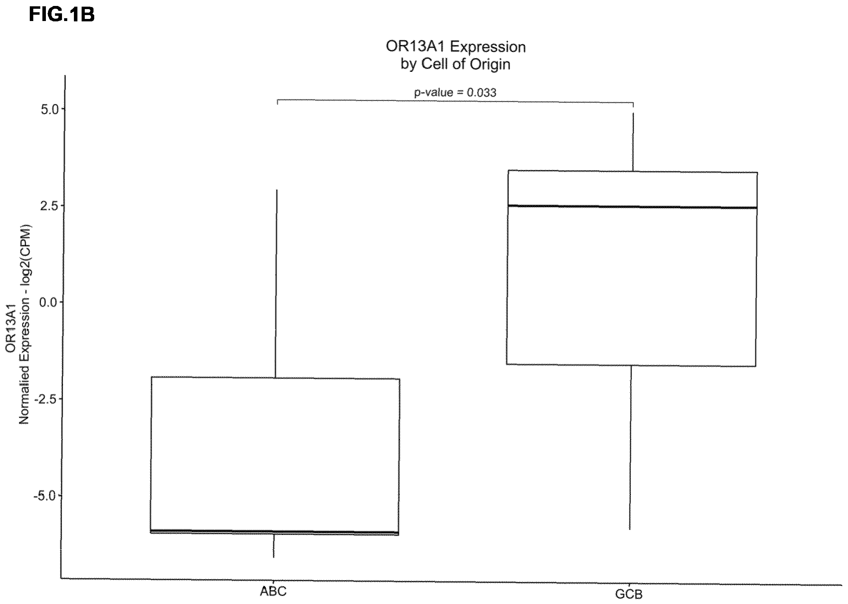

**Figure 1****. Expression of OR13A1 across lymphoma cell lines as measured by RNA-Seq.** A) Heatmap is based on log2 CPM (counts per million). Scale ranges from low (white) to high expression (black). Each horizontal represents the value for one cell line. GCB DLBCL, diffuse large B-cell lymphoma of the germinal center B-cell-type; ABC DLBCL, diffuse large B-cell lymphoma of the activated B-cell-like; MCL, mantle cell lymphoma; MZL, marginal zone lymphoma; CLL, chronic lymphocytic leukemia; PMBCL, primary mediastinal large B-cell lymphoma; BL, Burkitt lymphoma. B) OR13A1 expression level by cell of origin in DLBCL cell lines, the expression was higher in cells derived from germinal center (GC) B-cell type (GCB) than activated B cell like (ABC) DLBCL (P value = 0.03).

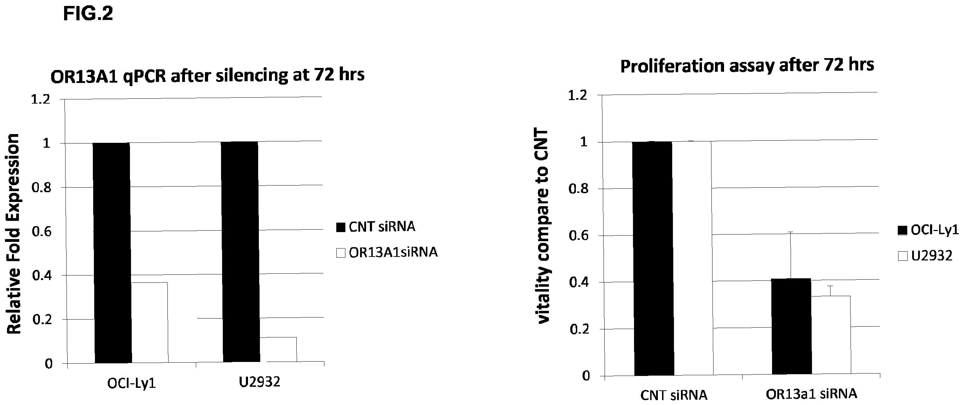

**Figure 2****. OR13A1 silencing is associated with reduction in cell viability of DLBCL cells.** Left panel, real-time PCR showing reduction of OR13A1 72h after siRNA treatment. Right panel, Reduction of cell viability, measured with MTT, in DLBCL cells after gene silencing.

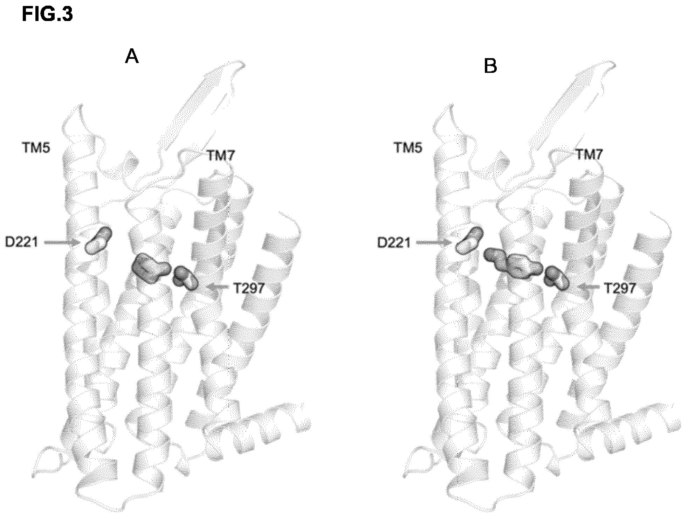

**Figure 3****. Homology model of OR13A1 with two ligands.** A) cyclohexanone docked onto the orthosteric binding site. B) tyramine docked onto the orthosteric binding site. Residues identified in the model to interact with the tyramine, potentially relevant for the receptor activation, are shown in sticks, D221 and T297.

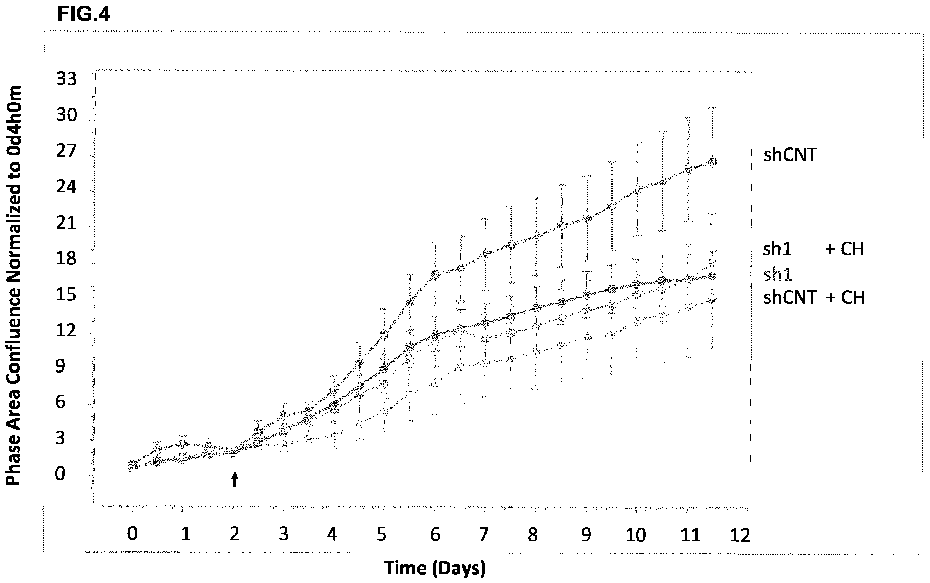

**Figure 4****. OR13A1 silencing and exposure to cyclohexanone reduces the growth of DLBCL cells.** The absence of OR13A1 abrogates the effect of cyclohexanone. Experiments were done using U2932 cells with the doxycycline-inducible shRNA system, exposed or not to cyclohexanone (25µM). The red arrow indicates the addition of cyclohexanone. CH, cyclohexanone; sh1, doxycycline-inducible shRNA targeting OR13A1; shCNT, scramble doxycycline-inducible shRNA.

### DETAILED DESCRIPTION OF THE INVENTION

The present invention is directed to the treatment or prevention of lymphomas by administration a modulator of a newly identified pathological target, the OR13A1 receptor.

In principle, all lymphomas can be target of the present treatment, i.e. those originated from B- or T-cell lymphoid cells, of Hodgkin's or non-Hodgkin's type, as well as multiple myeloma and immunoproliferative diseases as classified by the World Health Organizations under the term "lymphoma". In particular, based on the present experimental data, a higher efficacy is expected in the treatment of lymphomas from B-cell, in particular diffuse large B cells (DLBCL) and mantle cell lymphoma (MCL); Further based on the present data, the highest efficacy is expected in the treatment of the "germinal center B-cell" type of diffuse large B cells lymphoma (GCB-DLBCL); The GCB-DLBCL form of lymphoma is characterized by specific transcriptome and genetic features, as reported by Alizadeh AA, et al. Distinct types of diffuse large B-cell lymphoma identified by gene expression profiling. Nature. 2000 Feb 3;403(6769):503-11. doi: 10.1038/35000501. PMID: 10676951. Making available a medicament especially adapted to these forms of lymphoma represents therefore an important step in improving and optimizing the therapeutic tools for treating this disease.

Among lymphoma patients, preferred for the treatment are those with a high level of expression of the OR13A1 receptor; the level of expression of the OR13A1 receptor can evaluated for example via RNA-Seq and real-time PCR, or similar methods. A high level of expression is deemed to occur, according to the present invention, when this level, is significantly higher, e.g. at least 20% higher than the average level of expression in healthy patients;

A further object of the invention is a method of identifying a patient qualifiable for an improved treatment of lymphoma, in particular DLBCL or MCL, comprising the step of measuring the level of expression of OR13A1 protein in a patient cell; the method as claimed herein is performed *in vitro,* e.g. in a laboratory test tube, it is entirely performed outside the patient and does not include an intervention on the human body; the method has neither diagnostic role, being only performed to select best candidate patients for the present treatment, to whom a diagnosis of lymphoma has already been made prior to the present method. In the present method, patients having a high level of expression of the OR13A1 receptor will qualify as a candidates for the treatment; the level of expression of the OR13A1 receptor can evaluated by the aforementioned methods, A high level of expression is deemed to occur, according to the present invention, when this level, measured by the above referred method, is significantly higher, e.g. at least 20 % higher than the average level of expression in healthy patients. In a preferred embodiment, the method can be adapted to select best-responding candidate patients for the present treatment, i.e. those with lymphoma of the type GCB-DLBCL: these patients can be identified by the genetic characterization described by Alizadeh AA, et al. *op.cit.*

Any substance which acts as a modulator of the OR13A1 receptor can in principle be used in the frame of the present therapeutic uses and methods. Modulators of the OR13A1 receptor can be suitably identified by known techniques, e.g. receptor binding tests The modulators according to the invention, also referred herein as "ligands", can be agonists or antagonists of the OR13A1 receptor; alternatively, they can also be mixed-behaviour agents, i.e. partial OR13A1 antagonists with an agonist component or viceversa; preferably, the modulator is a OR13A1 receptor antagonist; a suitable experimental antagonist used in the present experiments was cyclohexanone. For the purpose of the invention, "antagonist" is defined as a compound having the capacity to inhibit at least one biological activity of the OR13A1 receptor; for example, an antagonist is characterized by a capacity to prevent the contraction of TM5 and TM7 towards the center of the bundle [26, 27], which is the initial step of the activation of GPCRs.

The invention includes a method of selecting further compounds useful for the treatment or prevention of lymphomas: the method includes the step of measuring the ligand activity of the candidate compound to the olfactory receptor OR13A1. The measurement of ligand activity can be performed as described above; any candidate compound showing a statistically significant ligand activity will be defined as selected compound. Among the lymphomas object of this method, DLBCL or MCL, in particular GCB-DLBCL can be mentioned.

For the purpose of the present invention, the terms "treatment" or "treating" define an approach for obtaining beneficial or desired clinical results. "Treatment" or "treating" covers any administration or application of a therapeutic for disease in a mammal, including humans. For purposes of this disclosure, beneficial or desired clinical results include, but are not limited to, any one or more of: alleviation of one or more symptoms, diminishment of extent of condition, preventing or delaying recurrence of condition, delay or slowing of condition progression, amelioration of the condition state, inhibiting the condition or progression of the condition, arresting development of the condition, and remission (whether partial or total) of the condition. Also encompassed by "treatment" or "treating" is a reduction of pathological consequence of a condition. The methods provided herein contemplate any one or more of these aspects of treatment. In-line with the above, the term treatment does not require one-hundred percent removal of all aspects of a condition or disorder.

Therapeutically effective amounts and dosing regimens will vary depending on a variety of factors including condition or disorder to be treated, as well as age, weight, possibly gender, and other health factors of the patient, which may be determined at the time of treatment. Therapeutically effective amounts may range from 1 mg to 500 mg. Dose units can contain, by way of non-limiting example, 1, 5, 10, 15, 20, 25, 30, 50, 75, 100, 150, 200, 250, 300, 350, 400, 450, or 500 mg of active ingredient as disclosed herein.

The present invention is now described by the following experimental examples. The examples are provided for illustration purpose, without having limitative functions; further embodiments involving modification of the present examples remain comprised in the invention.

### EXAMPLES

### Example 1

### G protein-coupled receptors of the olfactory receptors' family are expressed in lymphoma cell lines and clinical specimens

Using RNA-Seq and real-time PCR, few GPCRs of the olfactory receptors' family appeared expressed in lymphomas cell lines. OR13A1 was the most abundantly expressed compared to others olfactory receptors. Its expression was seen in 35/47 B cell lymphoma cell lines mostly derived from DLBCL and MCL. Among DLBCL cell lines, the expression was higher in cells derived from germinal center (GC) B-cell type (GCB) than activated B cell like (ABC) DLBCL (P value = 0.03) (cf. **Figure 1****).** This pattern observed in DLBCL cell lines can also be observed in patient's cells.

Other olfactory receptors were expressed in lymphoma cell lines. For example, 28 olfactory receptors were expressed in at least one cell line, including OR2B6, already mentioned for being expressed in breast cancer. Unsupervised clustering with these 28 transcripts largely divided cell lines based on their histology.

### Example 2

### G protein-coupled receptors of the olfactory receptors' family are essential for lymphoma cell lines

To obtain preliminary results on the potential biologic role of *OR13A1* in lymphomas, we have performed experiments using OCI-Ly1 and U2932, two cell lines derived from GCB and ABC DLBCL, respectively. Downregulation of *OR13A1* with siRNAs determined over 50% reduction in cell viability **(****Figure 2****),** and reduced phosphorylation of ERK, a known signaling event down-stream to olfactory receptors [21]. The viability data were confirmed using a doxycycline-inducible shRNA system and following the cells for 10 days with a live cell imaging system.

Finally, we have performed a CRISPR Cas9 screening using the genome-wide Brunello library against the MZL cell line VL51, parental or with acquired resistance to the combination of copanlisib/venetoclax. The experiments done in cells exposed to DMSO or to the combination has identified members of the olfactory receptor family 4 subfamily F (OR4F29, OR4F16, OR4F17, OR4F4, OR4F5) as essential for parental or resistant lymphoma cells, further highlighting the important biologic role played by this class of GPCRs.

### Example 3

### Modeling of G protein-coupled receptors of the olfactory receptors' family allows the identification of agonists and antagonists

Based on the previous data, we performed computer modelling. Human OR13A1 was modeled using the GPCR orexin receptor as a template (PDB code: 5wqc) [51]. In addition to human OR13A1, the two mouse receptors Olfr211 (orthologue to OR13A1) and Olfr1484, which were both experimentally demonstrated to have the natural compound cyclohexanone as ligand [52], were also modeled, using the same template. The three models were then used together to optimize the orthosteric binding site geometry of OR13A1. Next, we computationally docked the antagonist cyclohexanone in the binding site of the three proteins (**Figure 3A**). To ensure that the ligand is docked in the right position we exploited that, as an antagonist, cyclohexanone must prevent the contraction of TM5 and TM7 towards the center of the bundle [53, 54], the initial step of the activation of GPCRs. Finally, we used the OR13A1 and the binding pose cyclohexanone to identify an agonist of the receptor, tyramine (**Figure 3B**), exploiting the same mechanism, i.e., the contraction of TM5 and TM7 towards the center of the bundle [53, 54] .

In this way, we confirmed *in silico* the interaction of cyclohexanone with OR13A1, we predicted its activity as an antagonist. Moreover, we predicted other molecules (n.=30), structurally like cyclohexanone, to bind OR13A1 as well. Some, as tyramine, were predicted to act as agonists.

Cellular experiments followed with cyclohexanone and, among the predicted ligands, tyramine using the U2932 DLBCL cell line with the doxycycline-inducible shRNA system. The compound predicted to bind as agonist (tyramine) increased lymphoma cell line growth and the compound predicted to bind as antagonist (cyclohexanone) decreased the lymphoma cell viability in the presence but not in the absence of the GPCR **(****Figure 4****).**

The data presented in these examples show that OR13A1 gene is strongly and typically expressed in DLBCL and MCL as compared to others ORs, being found highest in GCB-DLBCL compared to ABC-DLBCL, thus having a most relevant therapeutic impact in the GCB form of DLBCL. The increase of tumour cell viability, reported in presence of OR13A1-expressing DLBCL, supports the inventor's finding that OR13A1 has a clear oncogenic role in lymphomas, in particular DLBCL and MCL, most particularly GCB-DLBCL; this is further highlighted by the increment of this activity in presence of the OR13A1 agonist tyramine; by converse, the anti-tumour utility of a suitable OR13A1 modulator is highlighted by the data of inhibition of tumour cell viability obtained by the standard antagonist cyclohexanone. The OR13A1 specificity of this mechanism is confirmed by the fact that no differences in tumour cell viability were observed in the present experiments when the receptor was silenced. Summarizing, the present genetic and chemical experiments support that OR13A1 has an essential function in lymphomas and represents a novel therapeutically responding target.

### REFERENCES

1. Siegel, R.L., et al., Cancer Statistics, 2021. CA Cancer J Clin, 2021. 71(1): p. 7-33.
2. Metzger, M.L. and C. Mauz-Körholz, Epidemiology, outcome, targeted agents and immunotherapy in adolescent and young adult non-Hodgkin and Hodgkin lymphoma. Br J Haematol, 2019. 185(6): p. 1142-1157.
3. Siegel, R.L., K.D. Miller, and A. Jemal, Cancer statistics, 2020. CA Cancer J Clin, 2020. 70(1): p. 7-30.
4. Roser, M. and H. Ritchie. Cancer. 2019; Available from: https://ourworldindata.org/cancer.
5. Howlader, N., et al., eds. SEER Cancer Statistics Review, 1975-2016, based on November 2018 SEER data submission, posted to the SEER web site, April 2019. 2019, National Cancer Institute: Bethesda, MD.
6. Sehn, L.H. and G. Salles, Diffuse Large B-Cell Lymphoma. N Engl J Med, 2021. 384(9): p. 842-858.
7. Silkenstedt, E., K. Linton, and M. Dreyling, Mantle cell lymphoma - advances in molecular biology, prognostication and treatment approaches. Br J Haematol, 2021. 195(2): p. 162-173.
8. Schöneberg, T. and I. Liebscher, Mutations in G Protein-Coupled Receptors: Mechanisms, Pathophysiology and Potential Therapeutic Approaches. Pharmacological Reviews, 2021. 73(1): p. 89-119.
9. Nugent, A. and R. L. Proia, The role of G protein-coupled receptors in lymphoid malignancies. Cellular Signalling, 2017. 39: p. 95-107.
10. Muppidi, J.R., et al., Loss of signalling via Galpha13 in germinal centre B-cell-derived lymphoma. Nature, 2014. 516(7530): p. 254-8.
11. Orecchioni, M., et al., Olfactory receptor 2 in vascular macrophages drives atherosclerosis by NLRP3-dependent IL-1 production. Science, 2022. 375(6577): p. 214-221.
12.Ichimura, A., et al., In silico approach to identify the expression of the undiscovered molecules from microarray public database: identification of odorant receptors expressed in non-olfactory tissues. Naunyn Schmiedebergs Arch Pharmacol, 2008. 377(2): p. 159-65.
13. Kang, N. and J. Koo, Olfactory receptors in non-chemosensory tissues. BMB Rep, 2012. 45(11): p. 612-22.
14. Flegel, C., et al., Expression profile of ectopic olfactory receptors determined by deep sequencing. PLoS One, 2013. 8(2): p. e55368.
15. Ranzani, M., et al., Revisiting olfactory receptors as putative drivers of cancer. Wellcome Open Res, 2017. 2(9): p. 9.
16. Weng, J., et al., PSGR2, a novel G-protein coupled receptor, is overexpressed in human prostate cancer. Int J Cancer, 2006. 118(6): p. 1471-80.
17. Bushdid, C., et al., Agonists of G-Protein-Coupled Odorant Receptors Are Predicted from Chemical Features. J Phys Chem Lett, 2018. 9(9): p. 2235-2240.
18. Massberg, D., et al., The activation of OR51E1 causes growth suppression of human prostate cancer cells. Oncotarget, 2016. 7(30): p. 48231-48249.
19.Masjedi, S., L.J. Zwiebel, and T.D. Giorgio, Olfactory receptor gene abundance in invasive breast carcinoma. Scientific Reports, 2019. 9(1): p. 13736.
20. Weber, L., et al., Olfactory Receptors as Biomarkers in Human Breast Carcinoma Tissues. Frontiers in oncology, 2018. 8: p. 33-33.
21. Li, M., et al., The Olfactory Receptor Family 2, Subfamily T, Member 6 (OR2T6) Is Involved in Breast Cancer Progression via Initiating Epithelial-Mesenchymal Transition and MAPK/ERK Pathway. Front Oncol, 2019. 9: p. 1210.
22. Gelis, L., et al., Functional Characterization of the Odorant Receptor 51E2 in Human Melanocytes. J Biol Chem, 2016. 291 (34): p. 17772-86.
23. Gelis, L., et al., Functional expression of olfactory receptors in human primary melanoma and melanoma metastasis. Exp Dermatol, 2017. 26(7): p. 569-576.
24. Weber, L., et al., Characterization of the Olfactory Receptor OR10H1 in Human Urinary Bladder Cancer. Front Physiol, 2018. 9: p. 456.
25. Abaffy, T., et al., A Testosterone Metabolite 19-Hydroxyandrostenedione Induces Neuroendocrine Trans-Differentiation of Prostate Cancer Cells via an Ectopic Olfactory Receptor. Front Oncol, 2018. 8: p. 162.
26. Novak, U., et al., The NF-{kappa}B negative regulator TNFAIP3 (A20) is inactivated by somatic mutations and genomic deletions in marginal zone lymphomas. Blood, 2009. 113(20): p. 4918-21.
27. Rinaldi, A., et al., Genome-wide DNA profiling of marginal zone lymphomas identifies subtype-specific lesions with an impact on the clinical outcome. Blood, 2011. 117(5): p. 1595-604.
28. Compagno, M., et al., Mutations of multiple genes cause deregulation of NF-kappaB in diffuse large B-celllymphoma. Nature, 2009. 459(7247): p. 717-21.
29. Bonetti, P., et al., Deregulation of ETS1 and FLI1 contributes to the pathogenesis of diffuse large β-cell lymphoma. Blood, 2013. 122(13): p. 2233-41.
30. Boi, M., et al., PRDM1/BLIMP1 is commonly inactivated in anaplastic large T-cell lymphoma. Blood, 2013. 122(15): p. 2683-93.
31. Chigrinova, E., et al., Two main genetic pathways lead to the transformation of chronic lymphocytic leukemia to Richter syndrome. Blood, 2013. 122(15): p. 2673-82.
32. Arribas, A.J., et al., DNA methylation profiling identifies two splenic marginal zone lymphoma subgroups with different clinical and genetic features. Blood, 2015. 125(12): p. 1922-31.
33.Arribas, A.J., et al., Genome-wide promoter methylation of hairy cell leukemia. Blood Adv, 2019. 3(3): p. 384-396.
34. Ferrero, S., et al., KMT2D mutations and TP53 disruptions are poor prognostic biomarkers in mantle cell lymphoma receiving high-dose therapy: a FIL study. Haematologica, 2020. 105(6): p. 1604-1612.
35. Tarantelli, C., et al., The Bruton tyrosine kinase inhibitor zanubrutinib (BGB-3111) demonstrated synergies with other anti-lymphoma targeted agents. Haematologica, 2019. 104(7): p. e307-e309.
36. Tarantelli, C., et al., Copanlisib synergizes with conventional and targeted agents including venetoclax in B- and T-cell lymphoma models. Blood Adv, 2020. 4(5): p. 819-829.
37. Spriano, F., et al., Single and combined BTK and PI3Kdelta inhibition with acalabrutinib and ACP-319 in pre-clinical models of aggressive lymphomas. Br J Haematol, 2019. 187(5): p. 595-601.
38. Gaudio, E., et al., Targeting CD205 with the antibody drug conjugate MEN1309/OBT076 is an active new therapeutic strategy in lymphoma models. Haematologica, 2020. 105(11): p. 2584-2591.
39. Hicks, S.W., et al., The novel CD19-targeting antibody-drug conjugate huB4-DGN462 shows improved anti-tumor activity compared to SAR3419 in CD19-positive lymphoma and leukemia models. Haematologica, 2019. 104(8): p. 1633-1639.
40.Tarantelli, C., et al., BET bromodomain inhibitor birabresib in mantle cell lymphoma: in vivo activity and identification of novel combinations to overcome adaptive resistance. ESMO Open, 2018. 3(6): p. e000387.
41. Mensah, A.A., et al., Bromodomain and extra-terminal domain inhibition modulates the expression of pathologically relevant microRNAs in diffuse large B-celllymphoma. Haematologica, 2018. 103(12): p. 2049-2058.
42. Spriano, F., et al., The ETS Inhibitors YK-4-279 and TK-216 Are Novel Antilymphoma Agents. Clin Cancer Res, 2019. 25(16): p. 5167-5176.
43.Tarantelli, C., et al., PQR309 Is a Novel Dual PI3K/mTOR Inhibitor with Preclinical Antitumor Activity in Lymphomas as a Single Agent and in Combination Therapy. Clin Cancer Res, 2018. 24(1): p. 120-129.
44. Gaudio, E., et al., Combination of the MEK inhibitor pimasertib with BTK or PI3K-delta inhibitors is active in preclinical models of aggressive lymphomas. Ann Oncol, 2016. 27(6): p. 1123-8.
45. Boi, M., et al., The BET Bromodomain Inhibitor OTX015 Affects Pathogenetic Pathways in Preclinical B-cell Tumor Models and Synergizes with Targeted Drugs. Clin Cancer Res, 2015. 21(7): p. 1628-38.
46. Spriano, F., et al., Antitumor activity of the dual BET and CBP/EP300 inhibitor NEO2734. Blood Adv, 2020. 4(17): p. 4124-4135.
47. Mensah, A.A., et al., Study of the antilymphoma activity of pracinostat reveals different sensitivities of DLBCL cells to HDAC inhibitors. Blood Adv, 2021. 5(10): p. 2467-2480.
48. Tarantelli, C., et al., The Novel TORC1/2 Kinase Inhibitor PQR620 Has Anti-Tumor Activity in Lymphomas as a Single Agent and in Combination with Venetoclax. Cancers, 2019. 11(6): p. 775.
49.Agirre, X., et al., Long non-coding RNAs discriminate the stages and gene regulatory states of human humoral immune response. Nat Commun, 2019. 10(1): p. 821.
50. Ennishi, D., et al., Double-Hit Gene Expression Signature Defines a Distinct Subgroup of Germinal Center B-Cell-Like Diffuse Large B-Cell Lymphoma. J Clin Oncol, 2019. 37(3): p. 190-201.
51. Suno, R., et al., Crystal Structures of Human Orexin 2 Receptor Bound to the Subtype-Selective Antagonist EMPA. Structure, 2018. 26(1): p. 7-19.e5.
52. Kida, H., et al., Vapor detection and discrimination with a panel of odorant receptors. Nat Commun, 2018. 9(1): p. 4556.
53. Sounier, R., et al., Propagation of conformational changes during µ-opioid receptor activation. Nature, 2015. 524(7565): p. 375-8.
54. Dror, R.O., et al., Activation mechanism of the β2-adrenergic receptor. Proc Natl Acad Sci U S A, 2011. 108(46): p. 18684-9.

## Claims

1. Ligand of the olfactory receptor OR13A1 for use in the treatment or prevention of lymphomas in a patient in need thereof.

2. Ligand for use according to claim 1, wherein the ligand is a OR13A1 receptor antagonist.

3. Ligand for use according to claim 2, wherein the antagonist is cyclohexanone or a pharmaceutically acceptable derivative thereof.

4. Ligand for use according to any one of claims 1-3, wherein the lymphomas are selected from diffuse large B cell lymphoma (DLBCL) and mantle cell lymphoma (MCL).

5. Ligand for use according to claim 4, wherein the DLBCL is lymphoma is a diffuse large B cell lymphoma of the germinal center B cell type (GCB-DLBCL).

6. Method of selecting a compound useful for the treatment or prevention of lymphomas including the step of assaying its ligand activity of the olfactory receptor OR13A1.

7. Method according to claim 6 wherein the ligand activity is of antagonist type.

8. Method of identifying a patient eligible for an improved treatment of lymphoma, comprising the initial step of *in vitro* measuring the level of expression of OR13A1 in a patient's cell, wherein the lymphoma is as described in any one of claims 1-5.
